# FASCICULE DE BREVET EUROPEEN

(11) **EP 3 655 381 B1**
(45) Date de publication et mention de la délivrance du brevet: **25.08.2021**
(21) Numéro de dépôt: 18773789.5
(22) Date de dépôt: 16.07.2018
(51) Int. Cl.: C07C 17/20, C07C 17/25, C07C 21/18, B01J 23/26, B01J 27/132, B01J 35/10, B01J 37/26

(54) **PROCEDE DE PRODUCTION DU 2,3,3,3-TETRAFLUOROPROPENE**
VERFAHREN ZUR HERSTELLUNG VON 2,3,3,3-TETRAFLUORPROPEN
PROCESS FOR THE PRODUCTION OF 2,3,3,3-TETRAFLUOROPROPENE

(30) Priorité: 17.07.2017 FR 1756726
(43) Date de publication de la demande: 27.05.2020
(73) Titulaire: Arkema France, 92700 Colombes (FR)
(72) Inventeur: COLLIER, Bertrand, 21500 Montbard (FR); DEUR-BERT, Dominique, 69390 Charly (FR); PIGAMO, Anne, 69340 Francheville (FR); WENDLINGER, Laurent, 69510 Soucieu En Jarrest (FR)
(74) Mandataire: Arkema Patent
(86) Numéro de dépôt international: PCT/FR2018/051795
(87) Numéro de publication internationale: WO 2019/016456

(56) Documents cités:
- WO-A1-2016/001515
- US-A1- 2015 148 571

## Description

### Domaine technique

La présente invention concerne un procédé de production de composés organiques fluorés, de préférence un procédé de production d'oléfines fluorées. En particulier, la présente invention se rapporte à un procédé de production de 2,3,3,3-tétrafluoropropène.

### Arrière-plan technologique de l'invention

Les hydrocarbures halogénés, en particulier les hydrocarbures fluorés comme les hydrofluorooléfines, sont des composés qui ont une structure utile comme matériaux fonctionnels, solvants, réfrigérants, agents de gonflage et monomères pour polymères fonctionnels ou matériaux de départ pour de tels monomères. Des hydrofluorooléfines comme le 2,3,3,3-tétrafluoropropène (HFO-1234yf) attirent l'attention parce qu'elles offrent un comportement prometteur comme réfrigérants à faible potentiel de réchauffement global.

Les procédés de production de fluorooléfines sont habituellement effectués en présence d'une substance de départ telle qu'un alcane contenant du chlore ou un alcène contenant du chlore, et en présence d'un agent fluorant tel que le fluorure d'hydrogène. Ces procédés peuvent être effectués en phase gazeuse ou en phase liquide, en absence ou non de catalyseur.

Les procédés en phase gazeuse sont habituellement effectués en présence de catalyseurs, en particulier en présence de catalyseurs à base de chrome. US 2015/0148571 dévoile un procédé de production d'oléfine contenant du fluor où le catalyseur est de l'oxyde de chrome fortement cristallisé. WO 2005/037431 dévoile une composition catalytique contenant du chrome comprenant ZnCr₂O₄ et un oxyde de chrome α cristallin et son utilisation dans un procédé pour modifier la distribution de fluor dans un hydrocarbure halogéné ou pour incorporer du fluor dans un hydrocarbure saturé ou insaturé. WO 2007/019353 dévoile la fabrication de 1,1,1,3,3-pentafluoropropane et 1,1,1,2,3-pentafluoropropane à partir d'un halopropène de formule CX₃CCl=CClX en présence d'un oxyde de chrome α cristallin, où au moins 0,05 % des atomes de chrome dans le réseau de l'oxyde de chrome α sont remplacés par un cuivre divalent. WO 98/10862 dévoile un catalyseur de fluoration à base d'oxyde de chrome III, dans lequel l'oxyde de chrome III est au moins partiellement et peut contenir un atome de zinc ou un de ses composés. Le catalyseur a été utilisé dans un procédé de fabrication de HFC-134a. Un oxyde fluorochrome ayant une teneur en fluor d'au moins 30 % en poids est également utilisé comme catalyseur dans un procédé de production d'oléfines contenant du fluor comme dévoilé dans EP 2 223 906.

Il existe également un besoin pour des compositions catalytiques ayant une forte activité (conversion) et/ou sélectivité ainsi que pour des procédés chimiques industriels sur la durée de vie d'un catalyseur.

### Résumé de l'invention

La présente invention se rapporte à un procédé de production du 2,3,3,3-tetrafluoropropène en phase gazeuse comprenant les étapes :
i) fourniture d'une composition **A** comprenant 2-chloro-3,3,3-trifluoropropène et/ou 2,3-dichloro-1,1,1-trifluoropropane et/ou 2-chloro-1,1,1,2-tetrafluoropropane ou d'une composition **B** comprenant 1,1,1,2,2-pentafluoropropane et/ou 1,1,1,2,3-pentafluoropropane ;
ii) mise en contact de ladite composition **A** avec de l'acide fluorhydrique en présence d'une composition catalytique comprenant un catalyseur à base de chrome ou mise en contact de ladite composition **B** avec une composition catalytique comprenant un catalyseur à base de chrome pour produire une composition **C** comprenant 2,3,3,3-tetrafluoropropène caractérisé en ce que l'étape ii) est mise en œuvre à une température comprise entre 310°C et 450°C et en ce que la température de l'étape ii) est contrôlée de sorte à ne pas excéder 450°C ; et lorsque ledit catalyseur est désactivé, la température de l'étape ii) est augmentée par incrémentation de 0,5°C à 20°C à condition que la température n'excède pas 450°C.

La présente invention permet de prolonger la durée de vie du catalyseur et d'améliorer l'efficacité de la réaction en évitant la présence d'impuretés générant la formation de coke.

Selon un mode de réalisation préféré, la température de la réaction mise en œuvre à l'étape ii) est comprise entre 310°C et 420°C, avantageusement entre 310°C et 400°C, de préférence entre 310°C et 375°C, plus préférentiellement entre 310°C et 360°C, en particulier entre 330°C et 360°C.

Selon un mode de réalisation préféré, la température de la réaction mise en œuvre à l'étape ii) n'excède pas 420°C, avantageusement n'excède pas 400°C, de préférence n'excède pas 375°C, plus préférentiellement n'excède pas 360°C.

Selon un mode de réalisation préféré, la température de l'étape ii) est augmentée par incrémentation de 0,5°C à 15°C, avantageusement de 0,5°C à 10°C, de préférence de 1°C à 10°C, plus préférentiellement de 1°C à 8°C, en particulier de 3°C à 8°C.

Selon un mode de réalisation préféré, le catalyseur à base de chrome est un oxyfluorure de chrome ou un oxyde de chrome ou un fluorure de chrome.

Selon un mode de réalisation préféré, le catalyseur à base de chrome comprend également un co-catalyseur sélectionné parmi le groupe consistant en Ni, Zn, Co, Mn ou Mg, de préférence la teneur en co-catalyseur est comprise entre 0,01% et 10% sur base du poids total du catalyseur.

Selon un mode de réalisation préféré, l'étape ii) est mise en œuvre à une pression supérieure à 1,5 bara.

### Description détaillée de l'invention

Le demandeur a observé de manière surprenante que des températures élevées pendant la fluoration ou la déshydrofluoration des produits de départ utilisés pour la production de 2,3,3,3-tétrafluoropropène peut résulter en une instabilité et une désactivation du catalyseur utilisé dans le procédé, une diminution de la sélectivité de la réaction envers le composé d'intérêt, i.e. 2,3,3,3-tétrafluoropropène, et/ou une augmentation en produits indésirables ou en impuretés. Ainsi, la présente invention fournit un procédé pour contrôler la température de réaction de sorte à prolonger la durée de vie du catalyseur et améliorer ainsi l'efficacité de la réaction.

La présente invention se rapporte à un procédé de production du 2,3,3,3-tetrafluoropropène en phase gazeuse. Le procédé comprend une étape i) de fourniture d'une composition **A** comprenant 2-chloro-3,3,3-trifluoropropène et/ou 2,3-dichloro-1,1,1-trifluoropropane et/ou 2-chloro-1,1,1,2-tetrafluoropropane ou d'une composition **B** comprenant 1,1,1,2,2-pentafluoropropane et/ou 1,1,1,2,3-pentafluoropropane.

De préférence, la composition **A** comprend au moins 20% en poids de 2-chloro-3,3,3-trifluoropropène et/ou 2,3-dichloro-1,1,1-trifluoropropane et/ou 2-chloro-1,1,1,2-tetrafluoropropane sur base du poids total de composition **A,** plus préférentiellement au moins 30% en poids, en particulier au moins 40% en poids, plus particulièrement au moins 50% en poids, de manière privilégiée au moins 60% en poids de 2-chloro-3,3,3-trifluoropropène et/ou 2,3-dichloro-1,1,1-trifluoropropane et/ou 2-chloro-1,1,1,2-tetrafluoropropane sur base du poids total de composition **A.**

De préférence, la composition **B** comprend au moins 20% en poids de 1,1,1,2,2-pentafluoropropane et/ou 1,1,1,2,3-pentafluoropropane sur base du poids total de composition **B,** plus préférentiellement au moins 30% en poids, en particulier au moins 40% en poids, plus particulièrement au moins 50% en poids, de manière privilégiée au moins 60% en poids de 1,1,1,2,2-pentafluoropropane et/ou 1,1,1,2,3-pentafluoropropane sur base du poids total de composition **B.**

Le présent procédé comprend également une étape :
ii) mise en contact de ladite composition **A** avec de l'acide fluorhydrique en présence d'un catalyseur à base de chrome ou mise en contact de ladite composition **B** avec un catalyseur à base de chrome pour produire une composition **C** comprenant 2,3,3,3-tetrafluoropropène.

L'étape ii) peut être mise en œuvre à une température comprise entre 310°C et 449°C ou entre 310°C et 448°C ou entre 310°C et 447°C ou entre 310°C et 446°C ou entre 310°C et 445°C ou entre 310°C et 444°C ou entre 310°C et 443°C ou entre 310°C et 442°C ou entre 310°C et 441°C ou entre 310°C et 440°C ou entre 310°C et 439°C ou entre 310°C et 438°C ou entre 310°C et 437°C ou entre 310°C et 436°C ou entre 310°C et 435°C ou entre 310°C et 434°C ou entre 310°C et 433°C ou entre 310°C et 432°C ou entre 310°C et 431°C ou entre 310°C et 430°C ou entre 310°C et 429°C ou entre 310°C et 428°C ou entre 310°C et 427°C ou entre 310°C et 426°C, ou entre 310°C et 425°C ou entre 310°C et 424°C ou entre 310°C et 423°C ou entre 310°C et 422°C, ou entre 310°C et 421°C.

Selon un mode de réalisation préféré, la température de la réaction mise en œuvre à l'étape ii) est comprise entre 310°C et 420°C. Avantageusement, la température de la réaction mise en œuvre à l'étape ii) est comprise entre 310°C et 419°C ou entre 310°C et 418°C ou entre 310°C et 417°C ou entre 310°C et 416°C ou entre 310°C et 415°C ou entre 310°C et 414°C ou entre 310°C et 413°C ou entre 310°C et 412°C ou entre 310°C et 411°C ou entre 310°C et 410°C ou entre 310°C et 409°C ou entre 310°C et 408°C ou entre 310°C et 407°C ou entre 310°C et 406°C ou entre 310°C et 405°C ou entre 310°C et 404°C ou entre 310°C et 403°C ou entre 310°C et 402°C ou entre 310°C et 401°C ou entre 310°C et 400°C. De préférence, la température de la réaction mise en œuvre à l'étape ii) est comprise entre 310°C et 398°C ou entre 310°C et 396°C ou entre 310°C et 394°C ou entre 310°C et 392°C ou entre 310°C et 390°C ou entre 310°C et 388°C ou entre 310°C et 386°C ou entre 310°C et 384°C ou entre 310°C et 382°C ou entre 310°C et 380°C ou entre 310°C et 378°C ou entre 310°C et 376°C ou entre 310°C et 375°C. Plus préférentiellement, la température de la réaction mise en œuvre à l'étape ii) est comprise entre 310°C et 374°C ou 310°C et 372°C ou 310°C et 370°C ou 310°C et 368°C ou 310°C et 366°C ou 310°C et 364°C ou 310°C et 362°C ou 310°C et 360°C. En particulier, la température de la réaction mise en œuvre à l'étape ii) est comprise entre 311°C et 360°C ou entre 312°C et 360°C ou entre 313°C et 360°C ou entre 314°C et 360°C ou entre 315°C et 360°C ou entre 316°C et 360°C ou entre 317°C et 360°C ou entre 318°C et 360°C ou entre 319°C et 360°C ou entre 320°C et 360°C ou entre 321°C et 360°C ou entre 322°C et 360°C ou entre 323°C et 360°C ou entre 324°C et 360°C ou entre 325°C et 360°C ou entre 326°C et 360°C ou entre 327°C et 360°C ou entre 328°C et 360°C ou entre 329°C et 360°C ou entre 330°C et 360°C.

Avantageusement, la température de la réaction mise en œuvre à l'étape ii) n'excède pas 449°C, n'excède pas 448°C, n'excède pas 447°C, n'excède pas 446°C, n'excède pas 445°C, n'excède pas 444°C, n'excède pas 443°C, n'excède pas 442°C, n'excède pas 441°C, n'excède pas 440°C, n'excède pas 439°C, n'excède pas 438°C, n'excède pas 437°C, n'excède pas 436°C, n'excède pas 435°C, n'excède pas 434°C, n'excède pas 433°C, n'excède pas 432°C, n'excède pas 431°C, n'excède pas 430°C, n'excède pas 429°C, n'excède pas 428°C, n'excède pas 427°C, n'excède pas 426°C, n'excède pas 425°C, n'excède pas 424°C, n'excède pas 423°C, n'excède pas 422°C, n'excède pas 421°C.

De préférence, la température de la réaction mise en œuvre à l'étape ii) n'excède pas 420°C, n'excède pas 419°C, n'excède pas 418°C, n'excède pas 417°C, n'excède pas 416°C, n'excède pas 415°C, n'excède pas 414°C, n'excède pas 413°C, n'excède pas 412°C, n'excède pas 411°C, n'excède pas 410°C, n'excède pas 409°C, n'excède pas 408°C, n'excède pas 407°C, n'excède pas 406°C, n'excède pas 405°C, n'excède pas 404°C, n'excède pas 403°C, n'excède pas 402°C, n'excède pas 401°C, n'excède pas 400°C, n'excède pas 399°C, n'excède pas 398°C, n'excède pas 397°C, n'excède pas 396°C, n'excède pas 395°C, n'excède pas 394°C, n'excède pas 393°C, n'excède pas 392°C, n'excède pas 391°C, n'excède pas 390°C, n'excède pas 389°C, n'excède pas 388°C, n'excède pas 387°C, n'excède pas 386°C, n'excède pas 385°C, n'excède pas 384°C, n'excède pas 383°C, n'excède pas 382°C, n'excède pas 381°C, n'excède pas 380°C, n'excède pas 379°C, n'excède pas 378°C, n'excède pas 377°C, n'excède pas 376°C, n'excède pas 375°C, n'excède pas 374°C, n'excède pas 373°C, n'excède pas 372°C, n'excède pas 371°C, n'excède pas 370°C, n'excède pas 369°C, n'excède pas 368°C, n'excède pas 367°C, n'excède pas 366°C, n'excède pas 365°C, n'excède pas 364°C, n'excède pas 363°C, n'excède pas 362°C, n'excède pas 361°C ou n'excède pas 360°C.

Selon un mode de réalisation préféré, la température de l'étape ii) est augmentée par incrémentation de 0,5°C 0,6°C, 0,7°C, 0,8°C, 0,9°C, 1,0°C, 1,1°C, 1,2°C, 1,3°C, 1,4°C, 1,5°C, 1,6°C, 1,7°C, 1,8°C, 1,9°C, 2,0°C, 2,1°C, 2,2°C, 2,3°C, 2,4°C, 2,5°C, 2,6°C, 2,7°C, 2,8°C, 2,9°C, 3,0°C, 3,1°C, 3,2°C, 3,3°C, 3,4°C, 3,5°C, 3,6°C, 3,7°C, 3,8°C, 3,9°C, 4,0°C, 4,1°C, 4,2°C, 4,3°C, 4,4°C, 4,5°C, 4,6°C, 4,7°C, 4,8°C, 4,9°C, 5,0°C, 5,1°C, 5,2°C, 5,3°C, 5,4°C, 5,5°C, 5,6°C, 5,7°C, 5,8°C, 5,9°C, 6,0°C, 6,1°C, 6,2°C, 6,3°C, 6,4°C, 6,5°C, 6,6°C, 6,7°C, 6,8°C, 6,9°C, 7,0°C, 7,1°C, 7,2°C, 7,3°C, 7,4°C, 7,5°C, 7,6°C, 7,7°C, 7,8°C, 7,9°C, 8,0°C, 8,1°C, 8,2°C, 8,3°C, 8,4°C, 8,5°C, 8,6°C, 8,7°C, 8,8°C, 8,9°C, 9,0°C, 9,1°C, 9,2°C, 9,3°C, 9,4°C, 9,5°C, 9,6°C, 9,7°C, 9,8°C, 9,9°C, 10,0°C, 10,1°C, 10,2°C, 10,3°C, 10,4°C, 10,5°C, 10,6°C, 10,7°C, 10,8°C, 10,9°C, 11,0°C, 11,1°C, 11,2°C, 11,3°C, 11,4°C, 11,5°C, 11,6°C, 11,7°C, 11,8°C, 11,9°C, 12,0°C, 12,1°C, 12,2°C, 12,3°C, 12,4°C, 12,5°C, 12,6°C, 12,7°C, 12,8°C, 12,9°C, 13,0°C, 13,1°C, 13,2°C, 13,3°C, 13,4°C, 13,5°C, 13,6°C, 13,7°C, 13,8°C, 13,9°C, 14,0°C, 14,1°C, 14,2°C, 14,3°C, 14,4°C, 14,5°C, 14,6°C, 14,7°C, 14,8°C, 14,9°C, 15,0°C, 15,1°C, 15,2°C, 15,3°C, 15,4°C, 15,5°C, 15,6°C, 15,7°C, 15,8°C, 15,9°C, 16,0°C, 16,1°C, 16,2°C, 16,3°C, 16,4°C, 16,5°C, 16,6°C, 16,7°C, 16,8°C, 16,9°C, 17,0°C, 17,1°C, 17,2°C, 17,3°C, 17,4°C, 17,5°C, 17,6°C, 17,7°C, 17,8°C, 17,9°C, 18,0°C, 18,1°C, 18,2°C, 18,3°C, 18,4°C, 18,5°C, 18,6°C, 18,7°C, 18,8°C, 18,9°C, 19,0°C, 19,1°C, 19,2°C, 19,3°C, 19,4°C, 19,5°C, 19,6°C, 19,7°C, 19,8°C, 19,9°C ou de 20,0°C.

Selon un mode de réalisation préféré, la température de l'étape ii) est augmentée par incrémentation de 0,5°C à 19°C, de 0,5°C à 18°C, de 0,5°C à 17°C, de 0,5°C à 16°C ou de 0,5°C à 15°C. Avantageusement, la température de l'étape ii) est augmentée par incrémentation de 0,5°C à 14°C, de 0,5°C à 13°C, de 0,5°C à 12°C, de 0,5°C à 11°C ou de 0,5°C à 10°C. De préférence, la température de l'étape ii) est augmentée par incrémentation de 0,6°C à 10°C, de 0,7°C à 10°C, de 0,8°C à 10°C, de 0,9°C à 10°C ou de 1°C à 10°C. Plus préférentiellement la température de l'étape ii) est augmentée par incrémentation de 1 à 9°C ou de 1°C à 8°C. En particulier la température de l'étape ii) est augmentée par incrémentation de 2°C à 8°C ou de 3°C à 8°C.

Dans ce procédé, la composition catalytique comprend un catalyseur à base de chrome. De préférence, le catalyseur à base de chrome peut être un oxyde de chrome (par exemple CrO₂, CrO₃ ou Cr₂O₃), un oxyfluorure de chrome ou un fluorure de chrome (par exemple CrF₃) ou un mélange de ceux-ci. L'oxyfluorure de chrome peut contenir une teneur en fluor comprise entre 1 et 60% en poids sur base du poids total de l'oxyfluorure de chrome, avantageusement entre 5 et 55% en poids, de préférence entre 10 et 52% en poids, plus préférentiellement entre 15 et 52% en poids, en particulier entre 20 et 50% en poids, plus particulièrement entre 25 et 45% en poids, de manière privilégiée entre 30 et 45% en poids, de manière plus privilégiée de 35 à 45% en poids de fluor sur base du poids total de l'oxyfluorure de chrome. La composition catalytique peut également comprendre un co-catalyseur choisi parmi le groupe consistant en Ni, Co, Zn, Mg, Mn, Fe, Zn, Ti, V, Zr, Mo, Ge, Sn, Pb, Sb; de préférence Ni, Co, Zn, Mg, Mn ; en particulier Ni, Co, Zn. La teneur en poids du co-catalyseur est comprise entre 1 et 10% en poids sur base du poids total de la composition catalytique. La composition catalytique peut également comprendre un support tel que l'alumine, par exemple sous sa forme alpha, de l'alumine activée, les halogénures d'aluminium (AlF₃ par exemple), les oxyhalogénures d'aluminium, du charbon actif, fluorure de magnésium ou du graphite. De préférence, la composition catalytique a une surface spécifique entre 1 et 100 m²/g, de préférence entre 5 et 80 m²/g, plus préférentiellement entre 5 et 70 m²/g, idéalement entre 5 et 50 m²/g, en particulier entre 10 et 50 m²/g, plus particulièrement entre 15 et 45 m²/g.

Selon un mode de réalisation préféré, l'étape ii) est mise en œuvre à la pression atmosphérique ou à une pression supérieure à celle-ci, avantageusement à une pression supérieure à 1,5 bara, de préférence à une pression supérieure à 2,0 bara, en particulier à une pression supérieure à 2,5 bara, plus particulièrement à une pression supérieure à 3,0 bara.

De préférence, l'étape ii) est mise en œuvre à une pression comprise entre la pression atmosphérique et 20 bara, de préférence entre 2 et 18 bara, plus préférentiellement entre 3 et 15 bara.

De préférence, l'étape ii) du présent procédé est mise en œuvre avec un temps de contact entre 1 et 100 s, de préférence entre 2 et 75 s, en particulier entre 3 et 50 s. Le procédé peut être mené sur une durée comprise entre 10 et 8000 h, de préférence entre 50 et 5000 h, plus préférentiellement entre 70 et 1000 h. On peut ajouter un oxydant, comme l'oxygène ou le chlore, en cours de procédé. Le rapport molaire de l'oxydant sur le composé hydrocarbure peut être entre 0,005 et 2, de préférence entre 0,01 et 1,5. L'oxydant peut être de l'oxygène pur, de l'air ou un mélange d'oxygène et d'azote.

Comme mentionné ci-dessus, la composition A est mise en contact avec de l'acide fluorhydrique en présence d'une composition catalytique telle que décrite ci-dessus. De préférence, le rapport molaire HF/composition A peut varier entre 1:1 et 150:1, de préférence entre 2:1 et 125:1, plus préférentiellement entre 3:1 et 100:1. Dans ce cas, le nombre de moles de l'ensemble des composés organiques constituant la composition A est pris en compte pour le calcul de ce rapport molaire. En particulier, le rapport molaire HF/2-chloro-3,3,3-trifluoropropène et/ou 2,3-dichloro-1,1,1-trifluoropropane et/ou 2-chloro-1,1,1,2-tetrafluoropropane peut varier entre 1:1 et 150:1, de préférence entre 2:1 et 125:1, plus préférentiellement entre 3:1 et 100:1.

De l'acide chlorhydrique peut être produit lors de la mise en œuvre de l'étape ii) avec la composition A. La composition C peut comprendre, outre 2,3,3,3-tétrafluoropropène, HCl, du HF n'ayant pas réagi et optionnellement du 2-chloro-3,3,3-trifluoropropène et/ou 2,3-dichloro-1,1,1-trifluoropropane et/ou 2-chloro-1,1,1,2-tetrafluoropropane n'ayant pas réagi. La composition C peut également comprendre 1,1,1,2,2-pentafluoropropane.

La composition **C** peut être purifiée, par exemple par distillation, dans des conditions efficaces pour former un courant **C1** comprenant HCl et 2,3,3,3-tétrafluoropropène et optionnellement 1,1,1,2,2-pentafluoropropane et un courant **C2** comprenant HF et 2-chloro-3,3,3-trifluoropropène et/ou 2,3-dichloro-1,1,1-trifluoropropane et/ou 2-chloro-1,1,1,2-tetrafluoropropane et optionnellement 1,1,1,2,2-pentafluoropropane. Alternativement, la composition **C** peut être purifiée, par exemple par distillation, dans des conditions efficaces pour former un courant **C1'** comprenant HCl et un courant **C2'** comprenant HF, 2,3,3,3-tétrafluoropropène et 2-chloro-3,3,3-trifluoropropène et/ou 2,3-dichloro-1,1,1-trifluoropropane et/ou 2-chloro-1,1,1,2-tetrafluoropropane et optionnellement 1,1,1,2,2-pentafluoropropane. Alternativement, la composition **C** peut être purifiée, par exemple par distillation, dans des conditions efficaces pour former un courant **C1"** comprenant HCl, 2,3,3,3-tétrafluoropropène et 2-chloro-3,3,3-trifluoropropène et/ou 2,3-dichloro-1,1,1-trifluoropropane et/ou 2-chloro-1,1,1,2-tetrafluoropropane et optionnellement 1,1,1,2,2-pentafluoropropane et un courant **C2"** comprenant HF.

Le courant **C1 ou** le courant **C1"** peut être distillé pour éliminer l'acide chlorhydrique et former une composition **C3** comprenant 2,3,3,3-tétrafluoropropène et optionnellement 1,1,1,2,2-pentafluoropropane ou une composition **C3"** comprenant 2,3,3,3-tétrafluoropropène et 2-chloro-3,3,3-trifluoropropène et/ou 2,3-dichloro-1,1,1-trifluoropropane et/ou 2-chloro-1,1,1,2-tetrafluoropropane et optionnellement 1,1,1,2,2-pentafluoropropane.

L'étape ii) peut être mise en œuvre avec la composition **B** avec ou sans acide fluorhydrique. Si de l'acide fluorhydrique est présent, le rapport molaire HF/composition **B** peut varier entre 1:1 et 150:1, de préférence entre 2:1 et 125:1, plus préférentiellement entre 3:1 et 100:1. Dans ce cas, le nombre de moles de l'ensemble des composés organiques constituant la composition **B** est pris en compte pour le calcul de ce rapport molaire. En particulier, le rapport molaire HF/1,1,1,2,2-pentafluoropropane et/ou 1,1,1,2,3-pentafluoropropane peut varier entre 1:1 et 150:1, de préférence entre 2:1 et 125:1, plus préférentiellement entre 3:1 et 100:1.

De préférence, de l'acide fluorhydrique est produit lors de la mise en œuvre de l'étape ii) avec la composition **B.** La composition **C** peut comprendre, outre 2,3,3,3-tétrafluoropropène, HF et optionnellement du 1,1,1,2,2-pentafluoropropane et/ou 1,1,1,2,3-pentafluoropropane n'ayant pas réagi.

La composition **C** peut être purifiée, par exemple par distillation, dans des conditions efficaces pour former un courant **C4** comprenant 1,1,1,2,2-pentafluoropropane et/ou 1,1,1,2,3-pentafluoropropane et 2,3,3,3-tétrafluoropropène et un courant **C5** comprenant HF. Alternativement, la composition **C** peut être purifiée, par exemple par distillation, dans des conditions efficaces pour former un courant **C4'** comprenant 2,3,3,3-tétrafluoropropène et un courant **C5'** comprenant HF et 1,1,1,2,2-pentafluoropropane et/ou 1,1,1,2,3-pentafluoropropane.

Un inhibiteur de polymérisation peut être utilisé pour améliorer la durée de vie du catalyseur, typiquement dans une concentration de 50 à 1000 ppm, de préférence de 100 à 500 ppm sur base du poids total de la composition **A** ou **B.** L'inhibiteur de polymérisation peut être p-methoxyphenol, t-amylphenol, limonène, d,l-limonène, quinones, hydroquinones, époxydes, amines, phosphates ou phosphorothionates ou des mélanges de ceux-ci.

### Exemple 1 (comparatif)

La fluoration du HCFO-1233xf en HFO-1234yf (2,3,3,3-tétrafluoropropène) et optionnellement en 1,1,1,2,2-pentafluoropropane est réalisée dans un réacteur multitubulaire avec un certain taux de conversion. Une boucle de recyclage dont le débit est contrôlé permet de ramener certains produits au réacteur de fluoration. Le réacteur contient un catalyseur massique à base d'oxyde de chrome. Le catalyseur est activé par une série d'étapes comprenant séchage, fluoration, traitement sous air et fluoration avec recyclage. Ce traitement en plusieurs étapes permet de rendre le solide catalytique actif et sélectif.

Le procédé de fluoration est mis en œuvre suivant les conditions opératoires suivantes :
- Une pression absolue dans le réacteur de fluoration de 6 bars absolu
- Un ratio molaire entre l'HF et la somme des organiques alimentés par la boucle de recyclage compris entre 11 et 13
- Un temps de contact compris entre 18 et 20 secondes
- Une température constante dans le réacteur de 350°C.

Le taux de conversion initial du HCFO-1233xf est supérieur à 72%. Un taux de conversion final de 41% est atteint après 616h de fonctionnement.

### Exemple 2

Le procédé selon l'invention est mis en œuvre de la même manière que l'exemple 1 avec les conditions opératoires suivantes :
- Une pression absolue dans le réacteur de fluoration de 6 bars absolu
- Un ratio molaire entre l'HF et la somme des organiques alimentés par la boucle de recyclage compris entre 11 et 13
- Un temps de contact compris entre 18 et 20 secondes
- Une température de départ dans le réacteur de 330°C puis une augmentation progressive de la température par incrémentation de 5°C :
   ο Au temps t = 151 h, la température du réacteur est fixée à 335°C,
   ο Au temps t = 323 h, la température du réacteur est fixée à 340°C,
   ο Au temps t = 457 h, la température du réacteur est fixée à 345°C,
   ο Au temps t = 599 h, la température du réacteur est fixée à 350°C.

Le taux de conversion initial du HCFO-1233xf est supérieur à 72%. Un taux de conversion final de 41% est atteint après 670h de fonctionnement, soit un gain de 10%.

## Revendications

1. Procédé de production du 2,3,3,3-tetrafluoropropène en phase gazeuse comprenant les étapes :
i) fourniture d'une composition **A** comprenant 2-chloro-3,3,3-trifluoropropène et/ou 2,3-dichloro-1,1,1-trifluoropropane et/ou 2-chloro-1,1,1,2-tetrafluoropropane ou d'une composition **B** comprenant 1,1,1,2,2-pentafluoropropane et/ou 1,1,1,2,3-pentafluoropropane ;
ii) mise en contact de ladite composition **A** avec de l'acide fluorhydrique en présence d'une composition catalytique comprenant un catalyseur à base de chrome ou mise en contact de ladite composition **B** avec une composition catalytique comprenant un catalyseur à base de chrome pour produire une composition **C** comprenant 2,3,3,3-tetrafluoropropène **caractérisé en ce que** l'étape ii) est mise en œuvre à une température comprise entre 310°C et 450°C et **en ce que** la température de l'étape ii) est contrôlée de sorte à ne pas excéder 450°C ; et lorsque ledit catalyseur est désactivé, la température de l'étape ii) est augmentée par incrémentation de 0,5°C à 20°C à condition que la température n'excède pas 450°C.

2. Procédé selon la revendication précédente **caractérisé en ce que** la température de la réaction mise en œuvre à l'étape ii) est comprise entre 310°C et 420°C, avantageusement entre 310°C et 400°C, de préférence entre 310°C et 375°C, plus préférentiellement entre 310°C et 360°C, en particulier entre 330°C et 360°C.

3. Procédé selon l'une quelconque des revendications précédentes **caractérisé en ce que** la température de la réaction mise en œuvre à l'étape ii) n'excède pas 420°C, avantageusement n'excède pas 400°C, de préférence n'excède pas 375°C, plus préférentiellement n'excède pas 360°C.

4. Procédé selon l'une quelconque des revendications précédentes **caractérisé en ce que** la température de l'étape ii) est augmentée par incrémentation de 0,5°C à 15°C, avantageusement de 0,5°C à 10°C, de préférence de 1°C à 10°C, plus préférentiellement de 1°C à 8°C, en particulier de 3°C à 8°C.

5. Procédé selon l'une quelconque des revendications précédentes **caractérisé en ce que** le catalyseur à base de chrome est un oxyfluorure de chrome ou un oxyde de chrome ou un fluorure de chrome.

6. Procédé selon l'une quelconque des revendications précédentes **caractérisé en ce que** le catalyseur à base de chrome comprend également un co-catalyseur sélectionné parmi le groupe consistant en Ni, Zn, Co, Mn ou Mg, de préférence la teneur en co-catalyseur est comprise entre 0,01% et 10% sur base du poids total du catalyseur.

7. Procédé selon l'une quelconque des revendications précédentes **caractérisé en ce que** l'étape ii) est mise en œuvre à une pression supérieure à 1,5 bara.

## Patentansprüche

1. Verfahren zur Herstellung von 2,3,3,3-Tetrafluorpropen in der Gasphase, das die folgenden Schritte umfasst:
i) Bereitstellen einer Zusammensetzung **A,** die 2-Chlor-3,3,3-trifluorpropen und/oder 2,3-Dichlor-1,1,1-trifluorpropan und/oder 2-Chlor-1,1,1,2-tetrafluorpropan umfasst, oder einer Zusammensetzung **B,** die 1,1,1,2,2-Pentafluorpropan und/oder 1,1,1,2,3-Pentafluorpropan umfasst;
ii) Inkontaktbringen der Zusammensetzung **A** mit Fluorwasserstoffsäure in Gegenwart einer Katalysatorzusammensetzung, die einen Katalysator auf Basis von Chrom umfasst, oder Inkontaktbringen der Zusammensetzung **B** mit einer Katalysatorzusammensetzung, die einen Katalysator auf Basis von Chrom umfasst, zur Herstellung einer Zusammensetzung **C,** die 2,3,3,3-Tetrafluorpropen umfasst, **dadurch gekennzeichnet, dass** Schritt ii) bei einer Temperatur zwischen 310 °C und 450 °C durchgeführt wird und dass die Temperatur in Schritt ii) derart gesteuert wird, dass sie 450 °C nicht übersteigt; und die Temperatur in Schritt ii) bei Deaktivierung des Katalysators schrittweise um 0,5 °C bis 20 °C erhöht wird, unter der Bedingung, dass die Temperatur 450 °C nicht übersteigt.

2. Verfahren nach dem vorhergehenden Anspruch, **dadurch gekennzeichnet, dass** die Temperatur der in Schritt ii) durchgeführten Reaktion zwischen 310 °C und 420 °C, vorteilhafterweise zwischen 310 °C und 400 °C, vorzugsweise zwischen 310 °C und 375 °C, weiter bevorzugt zwischen 310 °C und 360 °C, insbesondere zwischen 330 °C und 360 °C, liegt.

3. Verfahren nach einem der vorhergehenden Ansprüche, **dadurch gekennzeichnet, dass** die Temperatur der in Schritt ii) durchgeführten Reaktion 420 °C nicht übersteigt, vorteilhafterweise 400 °C nicht übersteigt, vorzugsweise 375 °C nicht übersteigt, weiter bevorzugt 360 °C, nicht übersteigt.

4. Verfahren nach einem der vorhergehenden Ansprüche, **dadurch gekennzeichnet, dass** die Temperatur in Schritt ii) schrittweise um 0,5 °C bis 15 °C, vorteilhafterweise um 0,5 °C bis 10 °C, vorzugsweise um 1 °C bis 10 °C, weiter bevorzugt um 1 °C bis 8 °C, insbesondere um 3 °C bis 8 °C, erhöht wird.

5. Verfahren nach einem der vorhergehenden Ansprüche, **dadurch gekennzeichnet, dass** es sich bei dem Katalysator auf Basis von Chrom um ein Chromoxidfluorid oder ein Chromoxid oder ein Chromfluorid handelt.

6. Verfahren nach einem der vorhergehenden Ansprüche, **dadurch gekennzeichnet, dass** der Katalysator auf Basis von Chrom auch einen aus der Gruppe bestehend aus Ni, Zn, Co, Mn oder Mg ausgewählten Cokatalysator umfasst, wobei der Gehalt an Cokatalysator zwischen 0,01 % und 10 %, bezogen auf das Gesamtgewicht des Katalysators, liegt.

7. Verfahren nach einem der vorhergehenden Ansprüche, **dadurch gekennzeichnet, dass** Schritt ii) bei einem Druck von mehr als 1,5 bara durchgeführt wird.

## Claims

1. Process for the gas-phase production of 2,3,3,3-tetrafluoropropene, comprising the steps:
i) providing a composition **A** comprising 2-chloro-3,3,3-trifluoropropene and/or 2,3-dichloro-1,1,1-trifluoropropane and/or 2-chloro-1,1,1,2-tetrafluoropropane or a composition **B** comprising 1, 1, 1, 2, 2-pentafluoropropane and/or 1,1,1,2,3-pentafluoropropane;
ii) placing said composition **A** in contact with hydrofluoric acid in the presence of a catalytic composition comprising a chromium-based catalyst or placing said composition **B** in contact with a catalytic composition comprising a chromium-based catalyst to produce a composition **C** comprising 2,3,3,3-tetrafluoropropene, **characterized in that** step ii) is performed at a temperature of between 310°C and 450°C and **in that** the temperature of step ii) is controlled so as not to exceed 450°C; and when said catalyst is deactivated, the temperature of step ii) is increased in increments of 0.5°C to 20°C on condition that the temperature does not exceed 450°C.

2. Process according to the preceding claim, **characterized in that** the reaction temperature used in step ii) is between 310°C and 420°C, advantageously between 310°C and 400°C, preferably between 310°C and 375°C, more preferentially between 310°C and 360°C, in particular between 330°C and 360°C.

3. Process according to either of the preceding claims, **characterized in that** the reaction temperature used in step ii) does not exceed 420°C, advantageously does not exceed 400°C, preferably does not exceed 375°C, more preferentially does not exceed 360°C.

4. Process according to any one of the preceding claims, **characterized in that** the temperature of step ii) is increased in increments of 0.5°C to 15°C, advantageously of 0.5°C to 10°C, preferably of 1°C to 10°C, more preferentially of 1°C to 8°C, in particular of 3°C to 8°C.

5. Process according to any one of the preceding claims, **characterized in that** the chromium-based catalyst is a chromium oxyfluoride or a chromium oxide or a chromium fluoride.

6. Process according to any one of the preceding claims, **characterized in that** the chromium-based catalyst also comprises a cocatalyst selected from the group consisting of Ni, Zn, Co, Mn and Mg; preferably, the content of cocatalyst is between 0.01% and 10% on the basis of the total weight of the catalyst.

7. Process according to any one of the preceding claims, **characterized in that** step ii) is performed at a pressure of greater than 1.5 bara.
